# EUROPEAN PATENT APPLICATION

(11) **EP 1 634 551 A2**
(43) Date of publication of application: **15.03.2006**
(21) Application number: 05108349.1
(22) Date of filing: 12.09.2005
(51) Int. Cl.: A61F 2/46

(54) **Surgical tool**

(30) Priority: 13.09.2004 GB 0420346
(71) Applicant: Finsbury (Development) Limited, Leatherhead, Surrey KT22 0BA (GB)
(72) Inventor: Wozencroft, Robert Michael, Epsom, Surrey KT19 8SS (GB)
(74) Representative: Smaggasgale, Gillian Helen

(57) **Abstract**

An alignment trial system for a hip prosthesis comprising: (a) a trial femoral prosthesis (2) comprising a head component (16), a neck component (4) and a stem component (6); and (b) a trial acetabular cup prosthesis (3) wherein the head component (16) of the trial femoral prosthesis (2) and the trial acetabular cup prosthesis (3) include interlocking engagement formations and wherein the trial acetabular cup prosthesis includes means to facilitate the formation of a bore in the pelvis.

## Description

The present invention relates to a tool for use in total hip replacement surgery resurfacing operations. More particularly, it relates to an acetabular trial system for use in total hip replacement surgery; a tool for positioning an acetabular cup and a method of carrying out total hip replacement surgery. Most particularly, it relates to a trial system which will assist the surgeon to determine the placement angles to enable the acetabular component of the hip prosthesis to be positioned at the optimal angle.

The efficient functioning of the hip joints is extremely important to the well being and mobility of the human body. Each hip joint is comprised by the upper portion of the femur which terminates in an offset bony neck surmounted by a ball-headed portion, known as the femoral head, which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component of the prosthesis can then be inserted. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modern prosthesis, the acetabular component may be coated on its external surface with a bone growth promoting substance which will assist the bone to grow and thereby assist the holding of the acetabular component in place. The bone femoral head will also be removed and the femur hollowed using reamers and rasps to accept the prosthesis. The stem portion is inserted into the femur.

In some cases, a femoral component of the kind described above may be replaced with components for use in femoral head resurfacing or for use in thrust plate technology.

In order to achieve optimal performance of the combined acetabular and femoral prosthesis, the acetabular cup must be properly positioned in the acetabulum. This is particularly important since incorrect positioning of the acetabular component can lead to the prosthetic hip joint suffering from dislocations, a decreased range of motion and possibly eventual loosening or failure of both components of the joint.

It is generally believed that there is an optimum orientation for the acetabular cup prosthesis to provide a full range of motion and to minimise the risk of dislocation. This orientation relative to the cup face is 45° to 50° from the vertical and is rotated forward to 15° to 20° of anteversion. This broadly replicates the natural angle of the acetabulum. However, the specific angle varies from patient to patient.

In hip replacement surgery, the cup portion of the prosthesis is usually orientated in the acetabulusn by using an acetabulum positioning instrument. Examples of these instruments generally include a horizontal arm that will be aligned parallel to the long axis of the patient at a preferred abduction angle. It is therefore important that the patient is correctly positioned on the operating table. The cup placement is typically done using a cup positioner and visually adjusting the cup to ensure that the horizontal arm of the positioner is approximately parallel to the long axis of the patient. The surgeon may also view the position relative to a second arm on the cup positioner which is positioned at a preset angle to position the cup at the correct abduction angle.

However, despite all care having been taken, the orientation of the cup in the replaced hip can deviate from the ideal. This may be due to one or more factors. First the positioning of the cup is judged by eye. As the position to be judged is a compound angle, it is particularly difficult to visualise. Second as the natural face of the acetabulum is not uniform and where the hip is arthritic may be distorted by osteophites, the acetabulum is not generally a reliable guide for orientating the cup implant. A third problem is that the prior art mechanical alignment guides usually rely on the pelvis being in a set position which may itself be difficult to judge particularly in an obese patient. In view of these difficulties, the cup may sometimes be located as much as about 20° from the optimum position.

With a view to overcoming these disadvantages, more high-tech arrangements have been suggested in which a CT-model of the bone structure is displayed on a computer screen and the surgeon is provided with information as to the positioning of the cup. However, arrangements of this type are not wholly satisfactory since they generally uses only a low number of measured landmark points to give the angle of the patient which may not provide the required level of accuracy. Further, the information provided by such systems may be difficult to interpret and may even provide the surgeon with a false sense of security Further these systems are generally expensive to install and operate and also in training costs.

Various proposals have been made in an attempt to overcome the problems associated with accurately locating the acetabular cup. In EP807426 an acetabular trial system is described which comprises an outer shell for placement on the acetabulum of a patient, an inner cup for placement within the outer shell which acts as a bearing, and a fixation device to rotatably fix the inner cup within the outer cup such that the inner cup can rotate about a selected axis. The appropriate anteversion angle of the acetabular cup of the hip prosthesis is determined automatically by placing the inner cup within the outer cup and providing a mechanism which will hold the inner cup in position after the surgeon has checked that the hip has the intended range of motion.

In US20031212459 an alternative trial shell is provided which enables a range of motion of the hip joint to be trialed before a prosthetic shell is implanted into the acetabular prosthesis.

Whilst these trial systems go some way to checking whether the selected position is correct, they still have problems in finding the correct position and thus there is still a requirement for an improved system which will enable a surgeon to insert an acetabular cup accurately.

The correct orientation of the stem of the femoral component is not generally difficult to achieve since the internal cavity of the proximal part of the femur will generally be based on the shape and axis of the bone and thus once the stem of the femoral prosthesis is placed in the bone it will generally be located at substantially the correct orientation. The correct orientation for a stem component is at 6° to 8° to the vertical and 20° to opposite anteversion i.e. opposite to the cup. The present invention is able to overcome the problems associated with prior art devices by making use of the accuracy of the placing of the femoral component.

The present invention makes use of the principle that with the hip joint connected (with the femoral head located in the acetabular cup) and in the proper position, the cup orientation can be derived from the stem orientation and vice versa. Since the optimum position of the stem can be readily achieved, the present invention relates to an alignment trial system for a hip prosthesis which enables the optimum position of the acetabular cup portion of the prosthesis to be derived from the position of the stem.

Thus according to the present invention there is provided an alignment trial system for a hip prosthesis comprising:
(a) a trial femoral prosthesis comprising a head component, a neck component and a stem component; and
(b) a trial acetabular cup prosthesis

wherein the head component of the trial femoral prosthesis and the trial acetabular cup prosthesis include interlocking engagement formations and wherein the trial acetabular cup prosthesis includes means to facilitate the formation of a bore in the pelvis.

Thus with the trial femoral stem located in a prepared femur and the trial acetabular cup located in a prepared pelvis, with the joint relocated and the engagement formations interlocked, the orientation of the cup in the pelvis can be marked such that the acetabular cup prosthesis can then be inserted accurately at the correct alignment.

The trial femoral prosthesis may be of any suitable configuration. It may be formed as a single piece or may be modular. A preferred arrangement is a modular trial femoral prosthesis which w-ill enable the surgeon to select a combination of components to provide a trial femoral prosthesis which most closely replicates the patient's femur in terms of neck offset, neck length and head size.

The modular trial femoral prosthesis may include two or more components. The modular trial femoral prosthesis will include at least a stem component and a head component. The head component may be provided with a neck the free end of which is attachable to the stem. Alternatively, the stem may include a neck, the free end of which is attachable to the head.

In a further embodiment, the modular trial femoral prosthesis may include a stem component, a head component and at least one neck component which is configured to connect to the head component and to the stem component.

In a particularly preferred arrangement, the modular trial femoral prosthesis includes a stem component, a head component and two neck components one of which is configured to connect to the head component and the other of which is configured to connect to the stem component; the free ends of the neck components being mutually connectable.

The components of the modular trial femoral prosthesis may connect together by any suitable means. In one arrangement, the connections between the components may be snap-fit connections.

The various components of a modular trial femoral prosthesis may include stop means to prevent or minimise any relative rotation of the components.

The engagement formation of the trial femoral prosthesis may be positioned such that it is suitable for use in one side of the body. Thus it will be suitable for use in only the left side or only the right side.

In an alternative arrangement, the head component of the trial femoral prosthesis may include two engagement formations for interlocking with the engagement formation in the trial acetabular cup; one being suitable for use if the alignment trial system is for use in the left side of the pelvis and the other being suitable for use if the alignment trial system is for use in the right side of the pelvis. Indicia may be included to identify the engagement formation that is suitable for use in the right side and that which is suitable for use in the left side.

The trial acetabular cup will usually include a single engagement formation. However, it will be understood that an arrangement may be provided where a single engagement formation is provided on the trial femoral prosthesis which would be suitable for use on either the left or right side and the cup may include two engagement formations for interlocking with the engagement formation in the trial acetabular cup; one being suitable for use if the alignment trial apparatus is for use in the left side of the pelvis and the other being suitable for use if the alignment trial apparatus is for use in the right side of the pelvis. Indicia may be included to identify the engagement formation that is suitable for use in the right side and that which is suitable for use in the left side.

The engagement means can be of any suitable configuration. They may comprise a male and female member which may be a sliding fit or may be a snap-fit. The engagement means may have a cross-section of any suitable shape. However, a circular cross section is generally preferred.

The means to facilitate the drilling of the bore may be of any suitable configuration. In one arrangement, a flange may extend from the rim of the trial acetabular cup having an aperture therein. Once the cup is in the correct position, the bore may be drilled through the aperture. Thus the bore will be a known distance and position from the cup and angled parallel to an axis passing through the centre of the trial acetabular cup prosthesis.

The foregoing arrangement is suitable where the acetabular cup prosthesis does not include a peg extending from the external surface of the prosthesis. In this arrangement, once the trial cup is removed, the acetabular cup prosthesis is brought into position using a surgical tool including a guidewire which is inserted into the bore drilled into the pelvis such that the acetabular cup prosthesis can be angled accurately. Since the guidewire will take the angle of the bore, it will cause the correct angle to be translated via the tool to the acetabular cup prosthesis.

In another aspect of the invention there is provided a surgical tool for gripping an acetabular cup prosthesis for implantation in a surgically prepared socket in a hip of a patient, the tool comprising an elongate body having a first end for releasable engagement with the prosthesis and a guidewire extending from the tool for insertion into a bore drilled into the pelvis. The insertion of the guide into the bore will ensure that the guidewire and hence the tool are at the correct angle to ensure that the prosthesis is placed at the optimal angle in the pelvis.

The guidewire generally extends along at least a portion of the tool. The guidewire may be connected to the tool by any suitable arrangement. It may pass through a sleeve in the side of the tool.

Where the acetabular cup prosthesis does include a peg a pin may be placed through the aperture in the flange to hold the trial cup in place while the remainder of the trial apparatus is removed, and a bore to accept the pin may be drilled into the pelvis through the base of the trial acetabular cup prosthesis. In this arrangement, the interlocking means in the cup will generally be a an annular collar around an aperture through which the drill may be introduced. Once the drill is removed, the pin and trial cup can be removed. It will be understood that as the bore is at the correct angle, the pin will be at the correct angle when inserted and will hold the acetabular cup prosthesis at the correct angle. In a more preferred arrangement, two flanges will be provided extending from the rim of the trial acetabular cup such that two pegs can be used to hold the cup in place during the drilling procedure to more firmly hold it in place. The flanges will generally be angled such that the pins are inserted in a converging manner as this will improve the holding of the cup. In this arrangement, a conventional tool may be used to introduce the cup.

The alignment trial apparatus of the present invention may be used in a method of inserting an acetabular cup prosthesis comprising the steps of:
(I) preparing the femur and pelvis for insertion of a femoral prosthesis and an acetabular cup prosthesis;
(ii) inserting the alignment trial apparatus of the above first aspect such that the trial femoral prosthesis is located in the prepared femur;
(iii) locating the patient's hip such that the trial cup prosthesis is seated in the prepared portion of the pelvis;
(v) drilling a bore in the pelvis;
(vi) removing the alignment trial apparatus;
(vii) inserting the acetabular cup prosthesis into the pelvis using the mark as a guide.

The location is preferably carried out with about 0° flexion of the hip i.e. with a straight leg.

The insertion of the acetabular cup is preferably inserted using the tool of the above second aspect wherein the mark is a well in the pelvis and the guidewire in the tool is inserted into the bore.

The present invention will now be described by way of example with reference to the following drawings:
- Figure 1: is an illustration of an alignment trial system in accordance with the present invention;
- Figure 2: illustrates the stem and a portion of the neck of a modular system;
- Figure 3: is an exploded view of the neck, head and cup components;
- Figure 4: is a perspective view from above of the cup and femoral trial components;
- Figure 5: is a view showing a drill being inserted to mark the pelvis;
- Figure 6: is a view illustrating the tool of the present invention; and
- Figure 7: illustrates an alternative trial cup

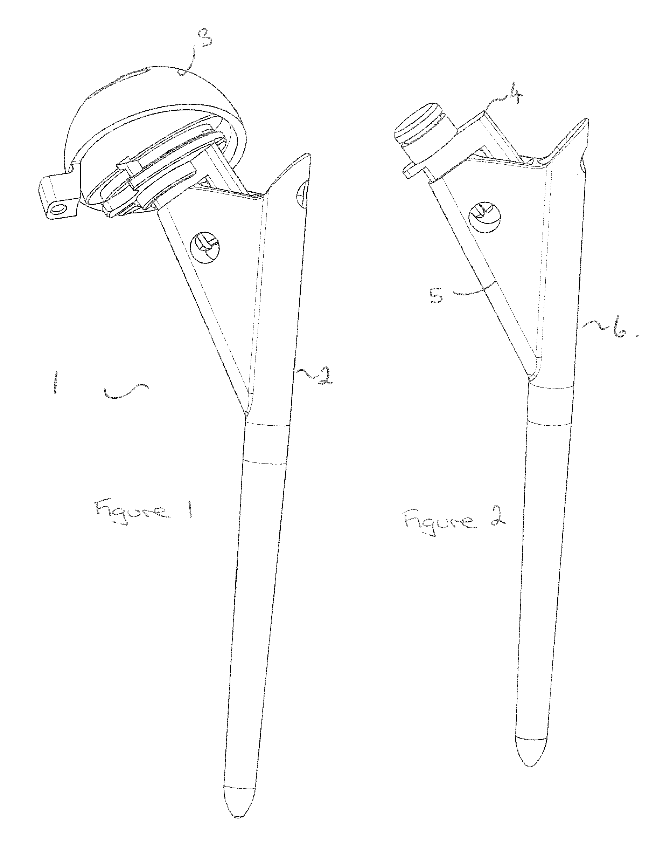

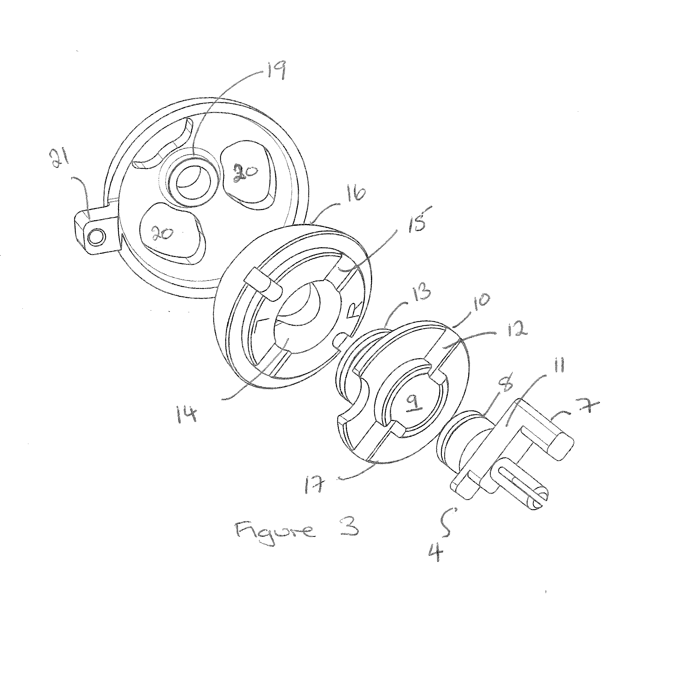

As illustrated in Figure 1, one arrangement of the alignment trial system 1 of the present invention comprises a trial femoral prosthesis 2 and a trial acetabular cup prosthesis 3. The trial femoral prosthesis is modular and two components are shown in Figure 2. Here a first neck component 4 is inserted in a side arm 5 of the stem component 6.

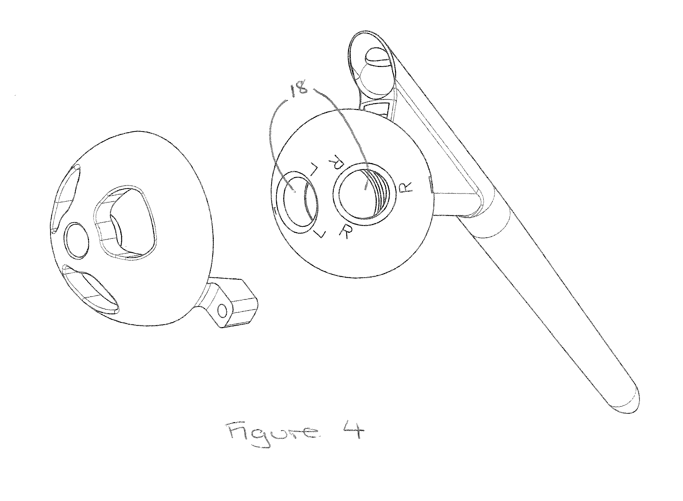

The first neck component 4 can be seen more clearly in Figure 3. It includes a flag 7 which when inserted between the walls of the side arm 5 and prevents the rotation of the component. A collar 8 extends upwardly therefrom to interlock with a corresponding aperture 9 in the second neck component 10. When the collar 8 is inserted into the hole 9 and locked in place, the arm 11 of the first neck component will sit in groove 12 such that mutual rotation of first and second neck components cannot occur. A collar 13 extends upwardly to interlock with the corresponding aperture 14 in the head component 16.

A groove 15 in the underside of the head component 16 will interlock with an arm (not shown) in the upper surface of plate 17 to prevent rotation. A portion of plate 17 may be removed to improve the line of sight to the aperture in the flange on the trial cup prosthesis which will be discussed in greater detail below.

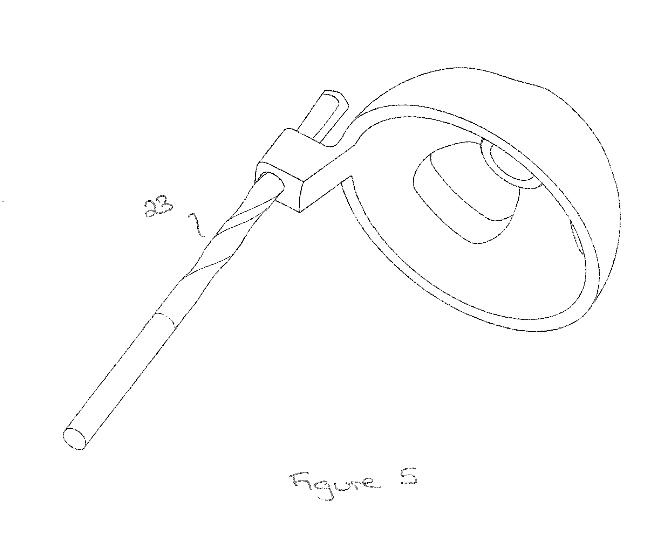

As illustrated in Figure 4 the head portion includes two apertures 18 one of which is suitable for engagement with the male member in the trial cup prosthesis if it is being used on the left and the other if the trial cup prosthesis is being used on the right.

The engagement means 19 in the cup prosthesis can be seen most clearly in Figure 3. This is a collar which is a snap-fit into the apertures 18. The spaces 20 in the acetabular cup prosthesis are provided to improve visibility of the pelvis as the trial cup is being seated.

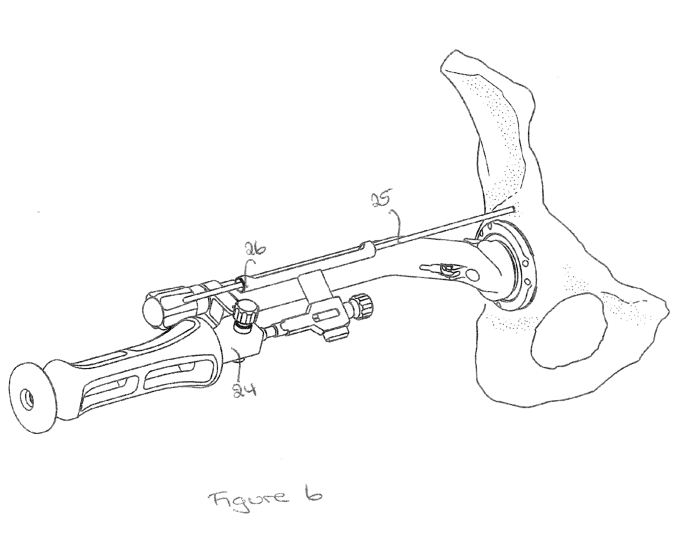

A flange 21 extends from the rim of the cup and has an aperture in 22 through which in use a drill bit 23 may be placed as illustrated in Figure 5.

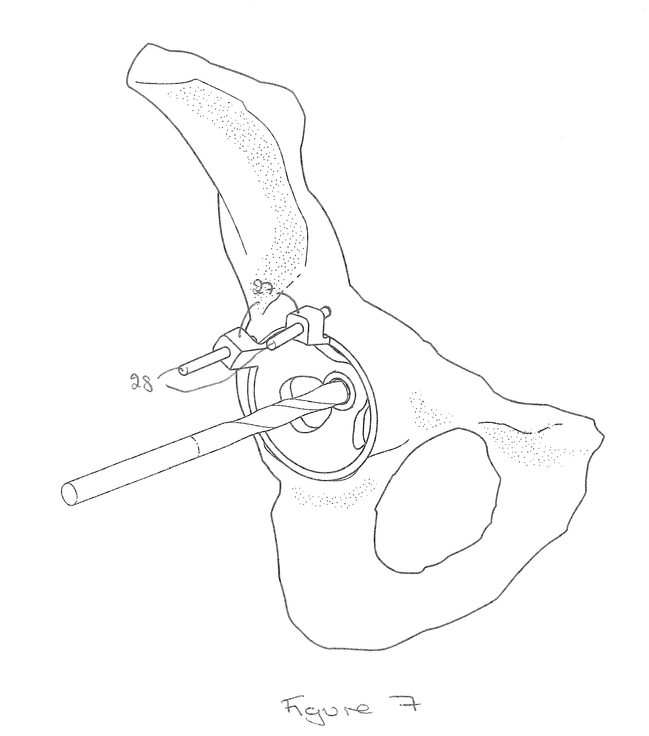

Figure 6 shows the use of the tool 24 of the present invention with the guidewire 25 passing through a collar 26 on the tool and into the bore in the pelvis.

Figure 7 illustrates an alternative arrangement where there are two flanges 27 to enable two pegs which can be driven into the pelvis to hold the cup in position while the drill is passed through the collar 19.

## Claims

1. An alignment trial system for a hip prosthesis comprising:
(a) a trial femoral prosthesis (2) comprising a head component (16), a neck component (4) and a stem component (6); and
(b) a trial acetabular cup prosthesis (3)
wherein the head component (16) of the trial femoral prosthesis (2) and the trial acetabular cup prosthesis (3) include interlocking engagement formations and
wherein the trial acetabular cup prosthesis includes means to facilitate the formation of a bore in the pelvis.

2. The alignment trial system according to Claim 1 wherein the trial femoral prosthesis is a single piece.

3. The alignment trial system according to Claim 1 wherein the trial femoral prosthesis is modular.

4. The alignment trial system according to Claim 3 wherein the modular trial femoral prosthesis includes two or more components.

5. The alignment trial system according to Claim 3 wherein the modular trial femoral prosthesis includes at least a stem component (6) and a head component (16).

6. The alignment trial system according to Claim 3 wherein the modular trial femoral prosthesis includes a stem component (6), a head component (16) and at least one neck component (4) which is configured to connect to the head component and to the stem component.

7. The alignment trial system according to Claim 6 wherein there are two neck components.

8. The alignment trial system according to any one of Claims 3 to 7 wherein connections between the components are snap-fit connections.

9. The alignment trial system according to Claim 8 wherein the components of the modular trial femoral prosthesis include stop means.

10. The alignment trial system according to any one of Claims 1 to 9 wherein the head component (16) of the trial femoral prosthesis (2) includes two engagement formations for interlocking with the engagement formation in the trial acetabular cup; one being suitable for use if the alignment trial system is for use in the left side of the pelvis and the other being suitable for use if the alignment trial system is for use in the right side of the pelvis.

11. The alignment trial system according to Claim 11 wherein indicia is included to identify the engagement formation that is suitable for use in the right side and that which is suitable for use in the left side.

12. The alignment trial system according to any one of Claims 1 to I 1 wherein the engagement means are a snap-fit.

13. The alignment trial system according to any one of Claims 1 to 12 wherein the means to facilitate the drilling of the bore comprises a flange (21) extending from a rim of the trial acetabular cup prosthesis having an aperture therein.

14. The alignment trial system according to any one of Claims 1 to 12 wherein the means to facilitate the drilling of the bore comprises two flanges (27) extending from a rim of the trial acetabular cup such that two pegs can be used to hold the cup in place during the drilling procedure.

15. A surgical tool for gripping an acetabular cup prosthesis for implantation in a surgically prepared socket in a hip of a patient, the tool comprising an elongate body having a first end for releasable engagement with the prosthesis and a guidewire extending from the tool for insertion into a bore drilled into the pelvis.

16. A surgical tool according to Claim 15 wherein the guidewire extends along at least a portion af the tool.
